# EUROPÄISCHE PATENTSCHRIFT

(11) **EP 3 695 790 B1**
(45) Veröffentlichungstag und Bekanntmachung des Hinweises auf die Patenterteilung: **25.08.2021**
(21) Anmeldenummer: 20155028.2
(22) Anmeldetag: 31.01.2020
(51) Int. Cl.: A61B 17/00, A61B 17/29

(54) **MEDIZINISCHES INSTRUMENT**
MEDICAL INSTRUMENT
INSTRUMENT MÉDICAL

(30) Priorität: 12.02.2019 DE 102019103489
(43) Veröffentlichungstag der Anmeldung: 19.08.2020
(73) Patentinhaber: Karl Storz SE & Co. KG, 78532 Tuttlingen (DE)
(72) Erfinder: Blase, Bastian, 13187 Berlin (DE); Albrecht, Simon, 10551 Berlin (DE)
(74) Vertreter: Hofmeister, Frank

(56) Entgegenhaltungen:
- EP-A1- 0 596 213
- DE-A1-102015 015 655
- DE-A1-102015 103 913
- US-A1- 2008 147 113
- US-A1- 2011 276 084

## Beschreibung

Die Erfindung betrifft ein medizinisches Instrument mit einem hohlen Schaft, an dessen proximalem Ende eine Handhabe angeordnet ist und an dessen distalem Ende ein Werkzeug mit zwei Maulteilen angeordnet ist, von denen mindestens ein Maulteil relativ zu dem anderen Maulteilen verschwenkbar ist, wobei ein das Werkzeug tragender distaler Endbereich des Schaftes als gegenüber der Längsachse des Schaftes abwinkelbare Werkzeugspitze ausgebildet ist sowie die Werkzeugspitze um die Längsachse des Schaftes bzw. um die Längsachse der Werkzeugspitze drehbar ist, wobei das Verdrehen der Werkzeugspitze um die Längsachse des Schaftes bzw. der Längsachse der Werkzeugspitze über ein in dem hohlen Schaft drehbar gelagertes erstes Betätigungselement erfolgt, das proximalseitig mit der Handhabe in Wirkverbindung steht und wobei das Abwinkeln der Werkzeugspitze über ein axialverschiebbar im hohlen Schaft gelagertes und proximalseitig mit der Handhabe in Wirkverbindung stehendes zweites Betätigungselement erfolgt und wobei das mindestens eine verschwenkbare Maulteil des Werkzeugs über ein axialverschiebbar in der Werkzeugspitze gelagertes Zug-/ Druckelement zwischen einer geschlossenen und einer geöffneten Position verstellbar ist, das proximalseitig mit der Handhabe in Wirkverbindung steht, wobei das Zug-/ Druckelement zum Betätigen des mindestens einen verschwenkbaren Maulteils exzentrisch parallel zur Längsachse des Schaftes bzw. der Längsachse der Werkzeugspitze in der Werkzeugspitze gelagert ist.

Medizinische Instrumente für die endoskopische Chirurgie weisen in der Regel einen hohlen Schaft auf, an dessen proximalem Ende eine Handhabe und an dessen distalem Ende ein Werkzeug aus zwei relativ zueinander bewegbaren Maulteilen angeordnet ist. Das als Greif-, Halte- und/oder Schneidinstrument ausgebildete Werkzeug ist über die Handhabe betätigbar. Um bei den häufig beengten Arbeitsverhältnissen mit dem Werkzeug einen möglichst großen Wirkungsbereich abdecken zu können, sind viele endoskopische Instrumente so ausgebildet, dass das Werkzeug gegenüber der Längsachse des Schaftes abwinkelbar ausgebildet ist sowie das Werkzeug um die Längsachse des Schaftes drehbar ist.

Derartige medizinische Instrumente sind aus der Praxis in unterschiedlichen konstruktiven Ausgestaltungen bekannt. Um die verschiedenen Freiheitsgrade der Werkzeugspitze relativ zum Schaft bewerkstelligen zu können, bedarf es häufig eines äußerst komplizierten und auch baulich großen Aufbaus der verschiedenen Gelenke und Kraftübertragungselemente.

Gattungsgemäße medizinische Instrumente sind aus der EP 0 596 213 A1 sowie der US 2011/276084 A1 bekannt. Bei diesen bekannten medizinischen Instrumenten liegen die Drehpunkte zum Verschwenken der Werkzeugspitze und die zugehörigen Zug-/Druckelemente eng beieinander, so dass es eines relativ großen Kraftaufwandes zum Verschwenken der Werkzeugspitze bedarf.

Weitere medizinische Instrumente sind beispielsweise aus der DE 103 14 828 B3 sowie der DE 10 2015 015 655 A1 bekannt. Diese bekannten Instrumente haben sich in der Praxis bewährt, jedoch sind diese aufgrund ihrer Bauart und Baugröße nicht für alle Anwendungsbereiche geeignet.

Für den Anwender und Patienten wünschenswert ist aber einerseits ein möglichst geringer Durchmesser des Instrumentenschaftes und andererseits eine möglichst kurze Werkzeugspitze, um ein Höchstmaß an Geschicklichkeit bei der Handhabung des Instruments zu gewährleisten.

Detaillösungen zum Bau kompakterer medizinischer Instrumente sind aus der DE 10 2015 103 913 A1 mit einer Welle zum Übertragen einer Rotationsbewegung sowie der US 2008/147113 A1 mit einem sichelförmigen Kulissenelement zum Abwinkeln der Werkzeugspitze bekannt.

Davon ausgehend liegt der Erfindung die **Aufgabe** zugrunde, ein medizinisches Instrument der eingangs genannten Art zu schaffen, das bei einfachem Aufbau und kompakter Bauweise die volle Beweglichkeit der Werkzeugspitze gewährleistet.

Die **Lösung** dieser Aufgabenstellung ist erfindungsgemäß dadurch gekennzeichnet, dass die Werkzeugspitze über ein Kulissenelement gegenüber der Längsachse des Schaftes abwinkelbar am distalen Ende des Schaftes gelagert ist und, dass die Werkzeugspitze proximalseitig über einen exzentrisch zur Längsachse des Schaftes bzw. der Längsachse der Werkzeugspitze angeordneten hohlen Zapfen um die Längsachse des Schaftes bzw. um die Längsachse der Werkzeugspitze verdrehbar in dem Kulissenelement gelagert ist.

Durch die exzentrische Verlagerung des Zug-/ Druckelements zum Betätigen des mindestens einen verschwenkbaren Maulteils ist es möglich für den Mechanismus zum Abwinkeln der Werkzeugspitze Platz zu schaffen, ohne den Durchmesser des Schaftes und/oder der Werkzeugspitze zu vergrößern.

Der exzentrisch zur Längsachse des Schaftes bzw. der Längsachse der Werkzeugspitze angeordnete hohle Zapfen dient gleichzeitig zur führenden Lagerung des exzentrisch in der Werkzeugspitze gelagerten Zug-/ Druckelements zum Betätigen des mindestens einen verschwenkbaren Maulteils.

Mit einer praktischen Ausführungsform der Erfindung wird vorgeschlagen, dass das distale Ende des Schaftes gabelförmig ausgebildet zwei parallele und voneinander beabstandete Stege aufweist.

Zur Abwinkelung der in dem Kulissenelement gelagerten Werkzeugspitze wird mit der Erfindung vorgeschlagen, dass das Kulissenelement über Lagerzapfen gegenüber der Längsachse des Schaftes abwinkelbar zwischen den beiden Stegen des distalen Endes des Schaftes gelagert ist, wobei die Lagerzapfen in korrespondierenden Lageraufnahmen der Stege gelagert sind.

Das zum Abwinkeln der Werkzeugspitze im Schaft gelagerte zweite Betätigungselement ist gemäß einer bevorzugten Ausführungsform der Erfindung als axial verschiebbares Zug-/ Druckelement ausgebildet, das seinerseits distalseitig am Kulissenelement gelagert ist, um beim Ausüben einer Zugbewegung nach proximal das Kulissenelement und damit auch die im Kulissenelement gelagerte Werkzeugspitze gegenüber der Längsachse des Schaftes abzuwinkeln.

Um ein zu starkes Abwinkeln der Werkzeugspitze gegenüber dem Schaft zu verhindern, wird mit der Erfindung vorgeschlagen, dass die Abwinklung der Werkzeugspitze gegenüber der Längsachse des Schaftes über mindestens ein Anschlagelement begrenzbar ist. Das mindestens eine Anschlagelement ist vorteilhafterweise als in mindestens einem Steg des distalen Endes des Schaftes gelagerter Zapfen ausgebildet, der in eine im Kulissenelement ausgebildete Führungsbahn eingreift.

Das zum Verdrehen der Werkzeugspitze um die Längsachse des Schaftes bzw. der Längsachse der Werkzeugspitze dienende erstes Betätigungselement ist gemäß einer praktischen Ausführungsform der Erfindung als verdrehbar im Schaft gelagerte Welle ausgebildet, die ihrerseits distalseitig mit der Werkzeugspitze gekoppelt ist, wobei die Welle über eine Verbindungshülse an der Werkzeugspitze festgelegt ist, um die Rotation der Welle möglichst spielfrei auf die Werkzeugspitze zu übertragen.

Weiterhin wird mit der Erfindung vorgeschlagen, dass das axialverschiebbar in der Werkzeugspitze gelagerte Zug-/ Druckelement zum Betätigen des mindestens einen verschwenkbaren Maulteils des Werkzeugs über eine verdrehbar im Schaft gelagerte Welle antreibbar ist, die proximalseitig mit der Handhabe gekoppelt ist.

Um die Rotationsbewegung der Welle in eine Axialbewegung des Zug-/ Druckelements umzuwandeln, ist die Welle erfindungsgemäß an ihrem distalen Ende mit einer Spindel gekoppelt, wobei die Spindel über ein Außengewinde mit einem Innengewinde im Zug-/ Druckelement in Wirkverbindung steht. Das Außengewinde der Spindel einerseits und das Innengewinde im Zug-/ Druckelement bewirken die Umwandlung der Rotationsbewegung in eine reine Axialbewegung.

Die Welle und die Spindel sind vorteilhafterweise über eine Verbindungshülse miteinander gekoppelt, um die Rotation der Welle möglichst spielfrei auf die Spindel zu übertragen.

Um die Anordnung der Wellen innerhalb des Schaftes möglichst platzsparend bewerkstelligen zu können, wird mit der Erfindung vorgeschlagen, dass die Welle zum Verdrehen der Werkzeugspitze sowie die Welle zur Betätigung des Zug-/ Druckelements koaxial zueinander im Schaft gelagert sind, wobei die Welle zum Verdrehen der Werkzeugspitze als äußere Welle und die Welle zum Betätigen des mindestens einen verschwenkbaren Maulteils als innere Welle ausgebildet ist.

Weiterhin wird mit der Erfindung vorgeschlagen, dass die Wellen als flexible Wellen ausgebildet sind.

Gemäß einer bevorzugten Ausführungsform der Erfindung wird vorgeschlagen, dass in einem distalen Endbereich des Schaftes ein in Richtung der Längsachse des Schaftes verlaufender und bis zum distalen Ende des Schaftes reichender Schlitz ausgebildet ist, wobei der Schlitz entlang der Seite des Schaftes ausgebildet ist, die der Richtung der Abwinklung der Werkzeugspitze gegenüber liegt, um ein radiales Austreten der flexiblen Wellen aus dem Schaft beim Abwinkeln der Werkzeugspitze zu ermöglichen. Die Ausbildung der Wellen als flexible Wellen ermöglicht beim Abwinkeln der Werkzeugspitze ein ausbeulendes Austreten der Wellen aus dem Schaft. Dieses Ausbeulen der Wellen ermöglicht eine besonders kompakte und kurze Bauweise der Werkzeugspitze, weil auf komplizierte und die Baulänge vergrößernde mechanische Umlenkmechanismen zur Übertragung der Rotation der Wellen hin zur Werkzeugspitze verzichtet werden kann. Da das seitliche Ausbeulen der Wellen aus dem Schaft erst im Operationsgebiet zur Anwendung kommt, wird der Durchmesser des medizinischen Instruments durch diese Ausgestaltung nicht vergrößert.

Schließlich wird mit der Erfindung vorgeschlagen, dass die Welle zum Verdrehen der Werkzeugspitze und die Spindel zum Betätigen des mindestens einen verschwenkbaren Maulteils des Werkzeugs über einen koaxial auf der Spindel angeordneten und radial nach außen wirkenden Spannring relativ zueinander entkoppelt sind.

Weitere Merkmale und Vorteile der Erfindung ergeben sich anhand der zugehörigen Zeichnungen, in denen ein Ausführungsbeispiel eines erfindungsgemäßen medizinischen Instruments nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen Instruments;
- Fig. 2: eine perspektivische Explosionszeichnung des Details II gemäß Fig. 1;
- Fig. 3: perspektivische Ansicht der Werkzeugspitze in drei verschiedenen Arbeitspositionen;
- Fig. 4: eine teilweise geschnittene perspektivische Ansicht des distalen Endes des Schaftes des erfindungsgemäßen medizinischen Instruments;
- Fig. 5: eine vergrößerte und teilweise freigeschnittene Detailansicht der Darstellung gemäß Fig. 4 mit abgewinkelter Werkzeugspitze;
- Fig. 6: eine teilweise Detailansicht der Darstellung gemäß Fig. 5, den Mechanismus zur Betätigung der Maulteile darstellend;
- Fig. 7: eine teilweise Detailansicht der Darstellung gemäß Fig. 5, den Mechanismus zur Rotation der Werkzeugspitze darstellend und
- Fig. 8: eine Seitenansicht des distalen Endes des Schaftes, die Werkzeugspitze in verschiedenen Arbeitspositionen darstellend.

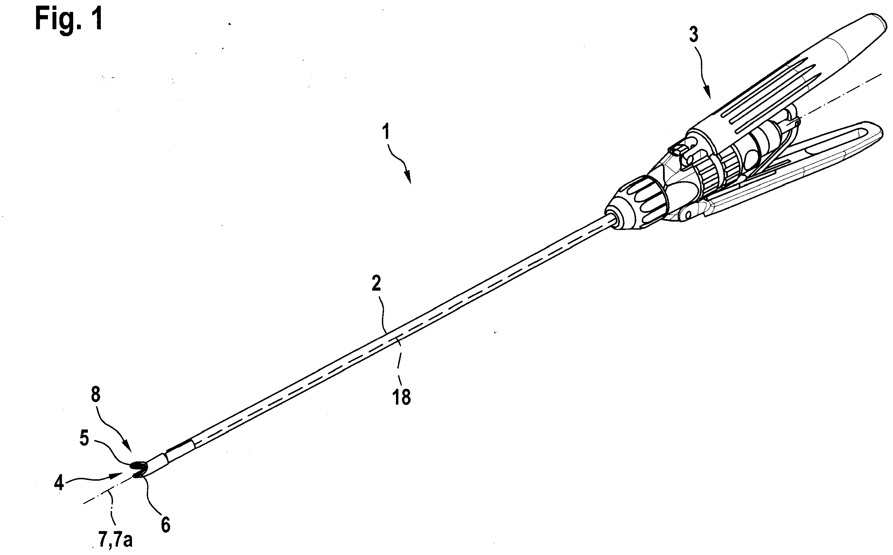

Die Abbildung Fig. 1 zeigt ein medizinisches Instrument 1 mit einem hohlen Schaft 2, an dessen proximalem Ende eine Handhabe 3 angeordnet ist und an dessen distalem Ende ein Werkzeug 4 angeordnet ist, das aus zwei Maulteilen 5 und 6 besteht, die bei der nachstehend näher beschriebenen Ausführungsform als relativ zueinander verschwenkbare Maulteile 5 und 6 ausgebildet sind.

Um dem Werkzeug 4 möglichst viele Freiheitsgrade zur Bewegung relativ zum Schaft 2 einzuräumen, ist ein das Werkzeug 4 tragender distaler Endbereich des Schaftes 2 als gegenüber der Längsachse 7 des Schaftes 2 abwinkelbare Werkzeugspitze 8 ausgebildet. Weiterhin ist das Werkzeug 4 um die Längsachse 7 des Schaftes 2 bzw. bei abgewinkelter Werkzeugspitze 8 um die Längsachse 7a der Werkzeugspitze 8 drehbar.

Zum Verschwenken der Maulteile 5 und 6 des Werkzeugs 4 relativ zueinander zwischen einer geschlossenen und einer geöffneten Position ist in der Werkzeugspitze 8 ein axial verschiebbares Zug-/ Druckelement 9 gelagert, das proximalseitig mit der Handhabe 3 in Wirkverbindung steht.

Das Verdrehen der Werkzeugspitze 8 um die Längsachse 7 des Schaftes 2 bzw. der Längsachse 7a der Werkzeugspitze 8 erfolgt über ein in dem hohlen Schaft 2 drehbar gelagertes erstes Betätigungselement 10, wohingegen das Abwinkeln der Werkzeugspitze 8 über ein axialverschiebbar im hohlen Schaft 2 gelagertes zweites Betätigungselement 11 erfolgt. Sowohl das erste Betätigungselement 10 zum Verdrehen der Werkzeugspitze 8 als auch das zweite Betätigungselement 11 zum Abwinkeln der Werkzeugspitze 8 stehen proximalseitig in Wirkverbindung mit der Handhabe 3.

Ein Hauptproblem bei der Herstellung derartiger medizinischer Instrumente 1 ist es, einerseits bei Beibehaltung aller Freiheitsgrade einen möglichst geringen Durchmesser des Schaftes 2 zu gewährleisten und andererseits die Werkzeugspitze 8 möglichst kurz auszugestalten, um ein Höchstmaß an Geschicklichkeit bei der Handhabung des medizinischen Instruments 1 zu gewährleisten.

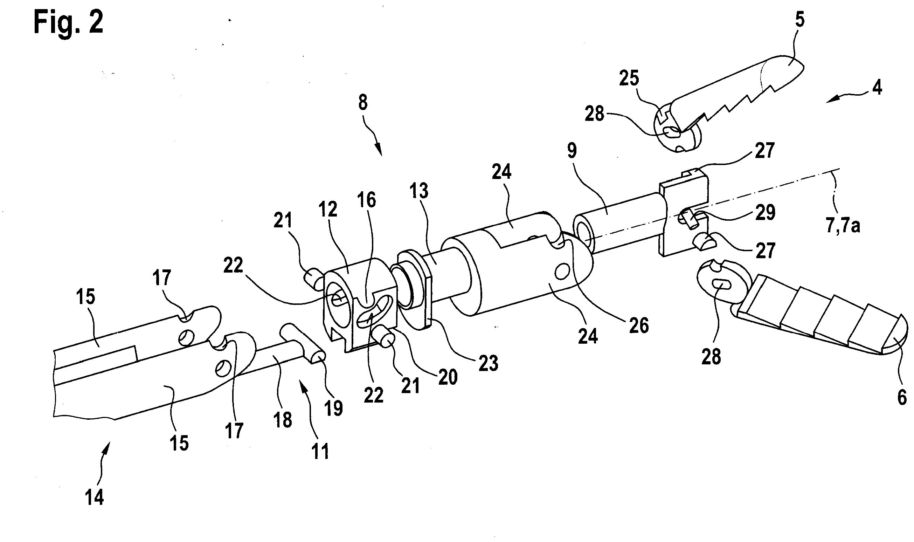

Um einerseits dem Zug-/ Druckelement 9 das Betätigen der verschwenkbaren Maulteile 5 und 6 zu ermöglichen und andererseits Platz für den Mechanismus zum Abwinkeln der Werkzeugspitze 8 zu schaffen, ohne den Durchmesser des Schaftes 2 und/oder der Werkzeugspitze 8 zu vergrößern, ist, wie insbesondere aus der Abbildung Fig. 2 ersichtlich, das Zug-/ Druckelement 9 zum Betätigen des der verschwenkbaren Maulteile 5 und 6 exzentrisch parallel zur Längsachse 7 des Schaftes 2 bzw. der Längsachse 7a der Werkzeugspitze 8 in der Werkzeugspitze 8 gelagert.

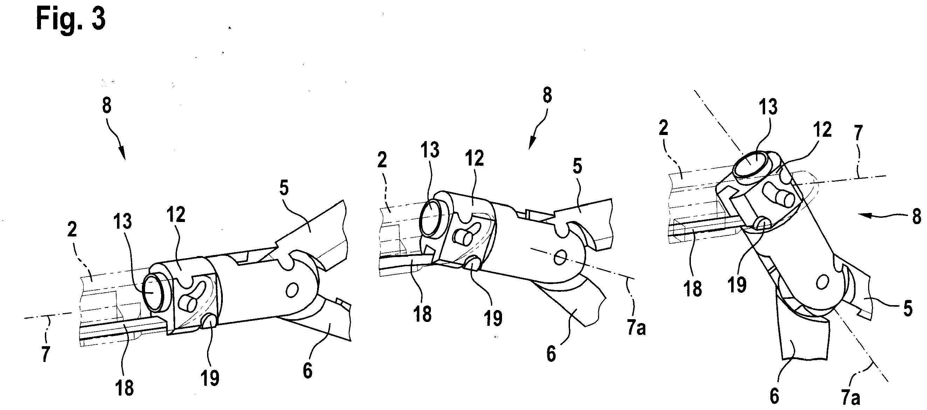

Wie aus einer Zusammenschau der Abbildungen Fig. 2 und 3 ersichtlich, ist die Werkzeugspitze über ein Kulissenelement 12 gegenüber der Längsachse 7 des Schaftes 2 abwinkelbar am distalen Ende des Schaftes 2 gelagert, wobei die Werkzeugspitze 8 proximalseitig über einen exzentrisch zur Längsachse 7 des Schaftes 2 bzw. der Längsachse 7a der Werkzeugspitze 8 angeordneten hohlen Zapfen 13 um die Längsachse 7 des Schaftes 2 bzw. um die Längsachse 7a der Werkzeugspitze 8 verdrehbar in dem Kulissenelement 12 gelagert ist. Der exzentrisch zur Längsachse 7 des Schaftes 2 bzw. der Längsachse 7a der Werkzeugspitze 8 angeordnete hohle Zapfen 13 dient gleichzeitig zur führenden Lagerung des exzentrisch in der Werkzeugspitze gelagerten Zug-/ Druckelements 9 zum Betätigen der verschwenkbaren Maulteile 5 und 6.

Zur Aufnahme des die Werkzeugspitze 8 tragenden Kulissenelements 12 weist das gabelförmig ausgebildete distale Ende 14 des Schaftes 2 zwei parallele und voneinander beabstandete Stege 15 auf.

Das Kulissenelement 12 selbst ist über Lagerzapfen 16 gegenüber der Längsachse 7 des Schaftes 2 abwinkelbar zwischen den beiden Stegen 15 des distalen Endes 14 des Schaftes 2 gelagert, wobei die Lagerzapfen 16 in korrespondierenden Lageraufnahmen 17 der Stege 15 gelagert sind.

Aufgrund der relativ weit voneinander beabstandeten exzentrischen Angriffspunkte der Lagerzapfen 16 und des zweiten Betätigungselements 11 am Kulissenelement 12 sinkt die zum Verschwenken der Werkzeugspitze 8 aufzubringende Kraft.

Zum Abwinkeln der Werkzeugspitze 8 ist, wie aus den Abbildungen Fig. 2 und 3 ersichtlich, das im Schaft 2 gelagerte zweite Betätigungselement 11 als axial verschiebbares Zug-/ Druckelement 18 ausgebildet, das distalseitig am Kulissenelement 12 gelagert ist. Das distale Ende des Zug-/ Druckelements 18 ist T-förmig so ausgebildet, dass der das distale Ende des Zug-/ Druckelements 18 bildende, quer zur Längsachse 7 des Schaftes 2 verlaufende Quersteg 19 des T-förmigen Zug-/ Druckelements 18 in einer Lageraufnahme 20 des Kulissenelements 12 gelagert ist, wobei der T-förmige Quersteg 19 vorteilhafterweise drehbar in der Lageraufnahme 20 gelagert ist, um bei einer Abwinklung der Werkzeugspitze 8 einen möglichen Winkelversatz ausgleichen zu können.

Beim Zurückziehen des Zug-/ Druckelements 18 nach proximal wird das über die Lagerzapfen 16 verschwenkbar in den Lageraufnahmen 17 der Stege 15 des distalen Endes 14 des Schaftes 2 gelagerte Kulissenelement 12 mitsamt der im Kulissenelement 12 gelagerten Werkzeugspitze 8 gegenüber der Längsachse 7 des Schaftes 2 verschwenkt, wie dies in der Abbildung Fig. 3 schematisch dargestellt ist.

Um beim Betätigen des Zug-/ Druckelements 18 ein zu starkes Abwinkeln der Werkzeugspitze 8 gegenüber dem Schaft 2 zu verhindern, ist die Abwinklung der Werkzeugspitze 8 gegenüber der Längsachse 7 des Schaftes 2 über mindestens ein Anschlagelement begrenzbar. Das mindestens eine Anschlagelement ist bei der dargestellten Ausführungsform als in mindestens einem Steg 15 des distalen Endes 14 des Schaftes 2 gelagerter Zapfen 21 ausgebildet, der in eine im Kulissenelement 12 ausgebildete Führungsbahn 22 eingreift.

Die Lagerung der Zapfen 21 in den Stegen 15 des distalen Endes 14 des Schaftes 2 sowie in den im Kulissenelement 12 ausgebildeten Führungsbahnen 22 verhindert zusätzlich, dass sich das Kulissenelement 12, insbesondere beim Abwinkeln der Werkzeugspitze 8, nach oben aus den Lageraufnahmen 17 herausheben kann.

Weiterhin umfasst der Mechanismus zum Verschwenken der Werkzeugspitze 8 eine Deckelscheibe 23, die distalseitig vor dem Kulissenelement 12 auf dem Zapfen 13 der Werkzeugspitze 8 gelagert ist und die Lageraufnahme 20 des Kulissenelements 12 bei eingesetztem Quersteg 19 des Zug-/ Druckelements 18 nach distal verschließt, um ein versehentliches Austreten des Querstegs 19 des Zug-/ Druckelements 18 aus der Lageraufnahme 20 zu verhindern.

Wie aus den Abbildungen Fig. 2 und 3 ersichtlich, ist es erst durch die parallele exzentrische Lagerung des Zug-/ Druckelements 9 zum Betätigen der Maulteile 5 und 6 möglich, ohne Vergrößerung des Außendurchmessers des Schaftes 2 und der Werkzeugspitze 8 auch den Mechanismus zum Verschwenken der Werkzeugspitze 8 im Übergangsbereich vom Schaft 2 zur Werkzeugspitze 8 auf engstem Raum anzuordnen.

Das Verstellen der verschwenkbaren Maulteile 5 und 6 des Werkzeugs 4 zwischen einer geschlossenen und eine geöffneten Position erfolgt, wie aus der Abbildung Fig. 2 ersichtlich, über das axialverschiebbar in der Werkzeugspitze 8 gelagerte Zug-/ Druckelement 9.

Zur Aufnahme des Zug-/ Druckelements 9 und zur Lagerung der verschwenkbaren Maulteile 5 und 6 weist das gabelförmig ausgebildete distale Ende der Werkzeugspitze 8 zwei parallele und voneinander beabstandete Stege 24 auf. Die Maulteile 5 und 6 weisen jeweils einen angeformten Lagerzapfen 25 auf, über den das jeweilige Maulteil 5 oder 6 verschwenkbar in einer korrespondierenden Lageraufnahme 26 in einem der Stege 24 gelagert ist. Die Kopplung der Maulteile 5 und 6 mit dem Zug-/ Druckelement 9 erfolgt über zwei am distalen Ende des Zug-/ Druckelements 9 ausgebildete Lagerzapfen 27, wobei jeder Lagerzapfen 27 jeweils in eine Aussparung 28 im proximalen Ende eines Maulteils 5 oder 6 eingreift.

Durch die ortsfeste Lagerung der Maulteile 5 und 6 an der Werkzeugspitze 8 über die in den Lageraufnahmen 26 angeordneten Lagerzapfen 25 der Maulteile 5 und 6 werden die Maulteile 5 und 6 beim axialen Verschieben des Zug-/ Druckelements 9 in Richtung der Längsachse 7a der Werkzeugspitze 8 zwischen einer geöffneten und einer geschlossenen Position relativ zueinander verschwenkt.

Der Bewegungsspielraum des Zug-/ Druckelements 9 in axialer Richtung nach distal wird durch einen zusätzlichen Stift 29 begrenzt, der ortsfest in den Stegen 24 der Werkzeugspitze 8 gelagert ist und gegen den das Zug-/ Druckelement 9 beim Verschieben in axialer Richtung als Anschlagelement anläuft.

Alternativ zu der, insbesondere in den Abbildungen Fig. 2 und 3, dargestellten Ausführungsform der Werkzeugspitze 8 und des Kulissenelements 12 können das proximale Ende des hohlen Zapfens 13 der Werkzeugspitze 8 und das proximale Ende des Kulissenelements 12 jeweils mit einer Fase als Anlaufschräge ausgebildet sein. Eine solche als Anlaufschräge dienende Fase bewirkt, dass beim Zurückziehen des medizinischen Instruments 1 in eine Trokarhülse die Werkzeugspitze 8 beim Anlaufen der Fase gegen die Innenwand der Trokarhülse wieder in eine vollständig gerade, in Richtung der Längsachse 7 des Schaftes 2 verlaufende Position überführt wird, falls die Werkzeugspitze 8 beim Einziehen des medizinischen Instruments 1 in die Trokarhülse noch ein wenig abgewinkelt war.

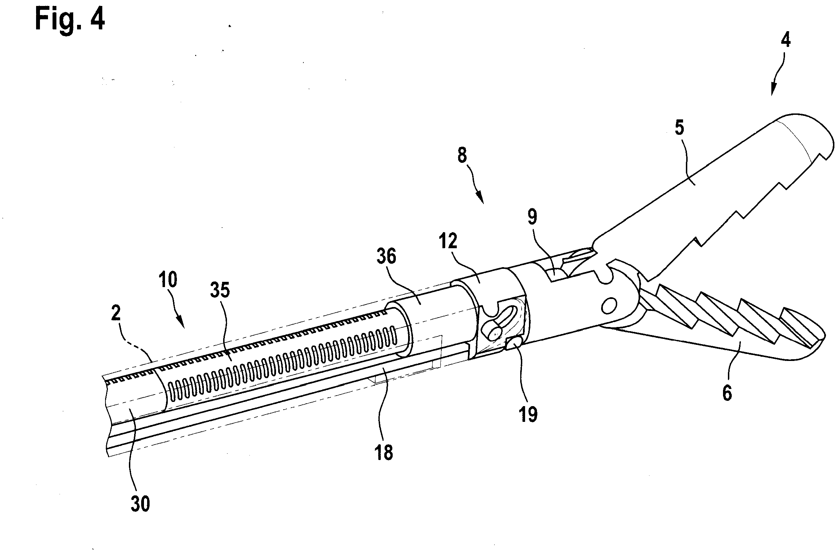

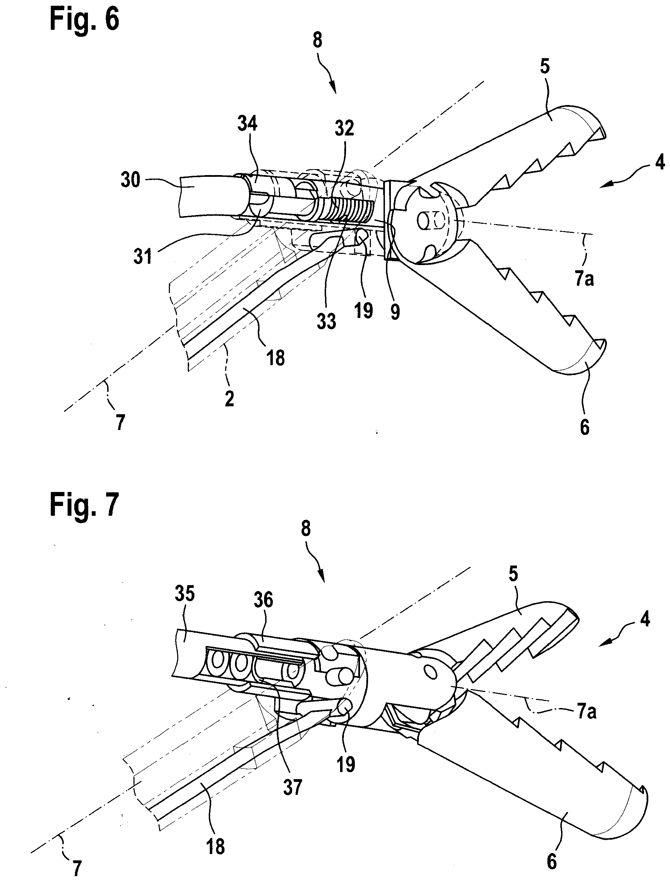

Die Kopplung des zum Betätigen der Maulteile 5 und 6 axial verschiebbar in der Werkzeugspitze 8 gelagerten Zug-/ Druckelements 9 mit der Handhabe 3 erfolgt über eine verdrehbar im Schaft 2 gelagerte Welle 30, die proximalseitig mit der Handhabe 3 gekoppelt ist und distalseitig mit dem Zug-/ Druckelement 9 in Wirkverbindung steht, wie dies den Abbildungen Fig. 4 bis 6 zu entnehmen ist.

Um die Rotationsbewegung der Welle 30 in eine Axialbewegung des Zug-/ Druckelements 9 umzuwandeln, ist die Welle 30 an ihrem distalen Ende mit einer Spindel 31 gekoppelt, wobei die Spindel 31 über ein Außengewinde 32 mit einem Innengewinde 33 im Zug-/ Druckelement 9 in Wirkverbindung steht. Das Außengewinde 32 der Spindel 31 einerseits und das Innengewinde 33 im Zug-/ Druckelement 9 andererseits bewirken die Umwandlung der Rotationsbewegung in eine reine Axialbewegung.

Die Welle 30 und die Spindel 31 sind vorteilhafterweise über eine Verbindungshülse 34 miteinander gekoppelt, um die Rotation der Welle 30 möglichst spielfrei auf die Spindel 31 zu übertragen.

Das zum Verdrehen der Werkzeugspitze 8 um die Längsachse 7 des Schaftes 2 bzw. der Längsachse 7a der Werkzeugspitze 8 dienende erste Betätigungselement 10 ist gemäß der dargestellten Ausführungsform als verdrehbar im Schaft 2 gelagerte Welle 35 ausgebildet, die ihrerseits distalseitig mit der Werkzeugspitze 8 gekoppelt ist, wobei Welle 35 über eine Verbindungshülse 36 an der Werkzeugspitze 8 festgelegt ist, um die Rotation der Welle 35 möglichst spielfrei auf die Werkzeugspitze 8 zu übertragen, wie dies den Abbildungen Fig. 4, 5 und 7 zu entnehmen ist.

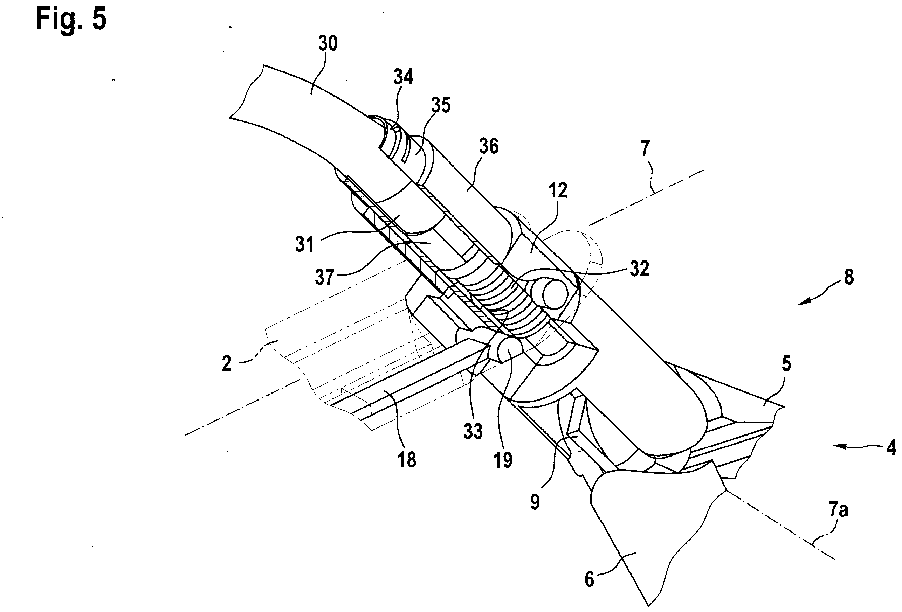

Um die Anordnung der Wellen 30 und 35 innerhalb des Schaftes 2 möglichst platzsparend bewerkstelligen zu können, sind die Welle 35 zum Verdrehen der Werkzeugspitze 8 sowie die Welle 30 zur Betätigung des Zug-/ Druckelements 9 koaxial zueinander im Schaft 2 gelagert, wobei die Welle 35 zum Verdrehen der Werkzeugspitze 8 als äußere Welle und die Welle 30 zum Betätigen der verschwenkbaren Maulteile 5 und 6 als innere Welle ausgebildet ist, wie dies den Abbildungen Fig. 4 und 5 zu entnehmen ist.

Wie aus Fig. 5 ersichtlich, ist am distalen Ende der Welle 35 zum Verdrehen der Werkzeugspitze 8 ein radial nach außen wirkender Spannring 37 koaxial auf der Spindel 31 angeordnet, um die Bewegung der Spindel 31 und der Welle 35 voneinander zu entkoppeln.

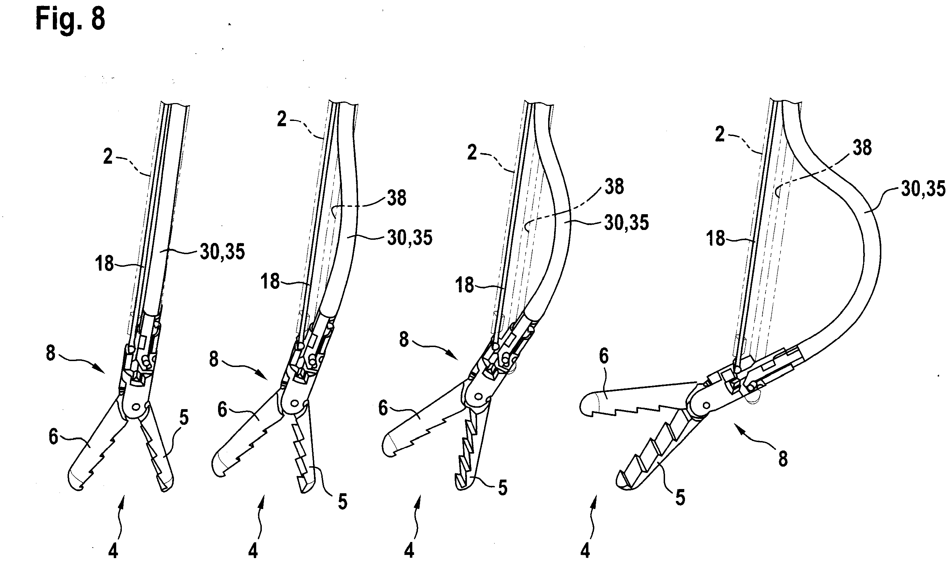

Die Abbildung Fig. 8 zeigt schließlich, dass im distalen Endbereich des Schaftes 2 ein in Richtung der Längsachse 7 des Schaftes 2 verlaufender und bis zum distalen Ende 14 des Schaftes 2 reichender Schlitz 38 ausgebildet ist. Dieser Schlitz 38 ist entlang der Seite des Schaftes 2 ausgebildet, die der Richtung der Abwinklung der Werkzeugspitze 8 gegenüber liegt, um beim Abwinkeln der Werkzeugspitze 8 ein radiales Austreten der flexiblen Wellen 30 und 35 aus dem Schaft 2 zu ermöglichen. Dieses Ausbeulen der Wellen 30 und 35 ermöglicht eine besonders kompakte und kurze Bauweise der Werkzeugspitze 8, weil auf komplizierte und die Baulänge vergrößernde mechanische Umlenkmechanismen zur Übertragung der Rotation der Wellen 30 und 35 hin zur Werkzeugspitze 8 verzichtet werden kann. Da das seitlich Ausbeulen der Wellen 30 und 35 aus dem Schaft 2 erst im Operationsgebiet zur Anwendung kommt, wird der Durchmesser des medizinischen Instruments 1 durch diese Ausgestaltung nicht vergrößert.

Ein wie zuvor beschrieben aufgebautes medizinisches Instrument 1 zeichnet sich dadurch aus, dass bei einfachem Aufbau und kompakter Bauweise die volle Beweglichkeit der Werkzeugspitze 8 gewährleistet ist.

### Bezugszeichenliste

- 1: medizinisches Instrument
- 2: Schaft
- 3: Handhabe
- 4: Werkzeug
- 5: Maulteil
- 6: Maulteil
- 7: Längsachse (Schaft)
- 7a: Längsachse (Werkzeugspitze)
- 8: Werkzeugspitze
- 9: Zug-/ Druckelement
- 10: erstes Betätigungselement
- 11: zweites Betätigungselement
- 12: Kulissenelement
- 13: Zapfen
- 14: distales Ende (Schaft)
- 15: Steg
- 16: Lagerzapfen
- 17: Lageraufnahme
- 18: Zug-/ Druckelement
- 19: Quersteg
- 20: Lageraufnahme
- 21: Zapfen (Anschlagelement)
- 22: Führungsbahn
- 23: Deckelscheibe
- 24: Steg
- 25: Lagerzapfen
- 26: Lageraufnahme
- 27: Lagerzapfen
- 28: Aussparung
- 29: Stift
- 30: Welle
- 31: Spindel
- 32: Außengewinde
- 33: Innengewinde
- 34: Verbindungshülse
- 35: Welle
- 36: Verbindungshülse
- 37: Spannring
- 38: Schlitz

## Patentansprüche

1. Medizinisches Instrument mit einem hohlen Schaft (2), an dessen proximalem Ende eine Handhabe (3) angeordnet ist und an dessen distalem Ende ein Werkzeug (4) mit zwei Maulteilen (5, 6) angeordnet ist, von denen mindestens ein Maulteil (5 oder 6) relativ zu dem anderen Maulteilen (6 oder 5) verschwenkbar ist, wobei ein das Werkzeug (4) tragender distaler Endbereich des Schaftes (2) als gegenüber der Längsachse (7) des Schaftes (2) abwinkelbare Werkzeugspitze (8) ausgebildet ist sowie die Werkzeugspitze (8) um die Längsachse (7) des Schaftes (2) bzw. um die Längsachse (7a) der Werkzeugspitze (8) drehbar ist, wobei das Verdrehen der Werkzeugspitze (8) um die Längsachse (7) des Schaftes (2) bzw. der Längsachse (7a) der Werkzeugspitze (8) über ein in dem hohlen Schaft (2) drehbar gelagertes erstes Betätigungselement (10) erfolgt, das proximalseitig mit der Handhabe (3) in Wirkverbindung steht und wobei das Abwinkeln der Werkzeugspitze (8) über ein axialverschiebbar im hohlen Schaft (2) gelagertes und proximalseitig mit der Handhabe (3) in Wirkverbindung stehendes zweites Betätigungselement (11) erfolgt und wobei das mindestens eine verschwenkbare Maulteil (5 oder 6) des Werkzeugs (4) über ein axialverschiebbar in der Werkzeugspitze (8) gelagertes Zug-/ Druckelement (9) zwischen einer geschlossenen und einer geöffneten Position verstellbar ist, das proximalseitig mit der Handhabe (3) in Wirkverbindung steht, wobei das Zug-/ Druckelement (9) zum Betätigen des mindestens einen verschwenkbaren Maulteils (5 oder 6) exzentrisch parallel zur Längsachse (7) des Schaftes (2) bzw. der Längsachse (7a) der Werkzeugspitze (8) in der Werkzeugspitze (8) gelagert ist,
**dadurch gekennzeichnet,**
**dass** die Werkzeugspitze (8) über ein Kulissenelement (12) gegenüber der Längsachse (7) des Schaftes (2) abwinkelbar am distalen Ende (14) des Schaftes (2) gelagert ist und, dass die Werkzeugspitze (8) proximalseitig über einen exzentrisch zur Längsachse (7) des Schaftes (2) bzw. der Längsachse (7a) der Werkzeugspitze (8) angeordneten hohlen Zapfen (13) um die Längsachse (7) des Schaftes (2) bzw. um die Längsachse (7a) der Werkzeugspitze (8) verdrehbar in dem Kulissenelement (12) gelagert ist.

2. Medizinisches Instrument nach Anspruch 1, **dadurch gekennzeichnet, dass** das distale Ende (14) des Schaftes (2) gabelförmig ausgebildet zwei parallele und voneinander beabstandete Stege (15) aufweist.

3. Medizinisches Instrument nach Anspruch 2, **dadurch gekennzeichnet, dass** das Kulissenelement (12) über Lagerzapfen (16) gegenüber der Längsachse (7) des Schaftes (2) abwinkelbar zwischen den beiden Stegen (15) des distalen Endes (14) des Schaftes (2) gelagert ist, wobei die Lagerzapfen (16) in korrespondierenden Lageraufnahmen (17) der Stege (15) gelagert sind.

4. Medizinisches Instrument nach einem der Ansprüche 1 bis 3, **dadurch gekennzeichnet, dass** das zum Abwinkeln der Werkzeugspitze (8) im Schaft (2) gelagerte zweite Betätigungselement (11) als axial verschiebbares Zug-/ Druckelement (18) ausgebildet ist, das distalseitig am Kulissenelement (12) gelagert ist.

5. Medizinisches Instrument nach einem der Ansprüche 1 bis 4, **dadurch gekennzeichnet, dass** die Abwinklung der Werkzeugspitze (8) gegenüber der Längsachse (7) des Schaftes (2) über mindestens ein Anschlagelement begrenzbar ist.

6. Medizinisches Instrument nach Anspruch 5, **dadurch gekennzeichnet, dass** das mindestens eine Anschlagelement als in mindestens einem Steg (15) des distalen Endes (14) des Schaftes (2) gelagerter Zapfen (21) ausgebildet ist, der in eine im Kulissenelement (12) ausgebildete Führungsbahn (22) eingreift.

7. Medizinisches Instrument nach einem der Ansprüche 1 bis 6, **dadurch gekennzeichnet, dass** das erstes Betätigungselement (10) zum Verdrehen der Werkzeugspitze (8) um die Längsachse (7) des Schaftes (2) bzw. der Längsachse (7a) der Werkzeugspitze (8) als verdrehbar im Schaft (2) gelagerte Welle (35) ausgebildet ist, die distalseitig mit der Werkzeugspitze (8) gekoppelt ist.

8. Medizinisches Instrument nach Anspruch 7, **dadurch gekennzeichnet, dass** die Welle (35) über eine Verbindungshülse (36) an der Werkzeugspitze (8) festgelegt ist.

9. Medizinisches Instrument nach einem der Ansprüche 1 bis 8, **dadurch gekennzeichnet, dass** das axialverschiebbar in der Werkzeugspitze (8) gelagerte Zug-/ Druckelement (9) zum Betätigen des mindestens einen verschwenkbaren Maulteils (5 oder 6) des Werkzeugs (4) über eine verdrehbar im Schaft (2) gelagerte Welle (30) antreibbar ist, die proximalseitig mit der Handhabe (3) gekoppelt ist.

10. Medizinisches Instrument nach Anspruch 9, **dadurch gekennzeichnet, dass** die Welle (30) an ihrem distalen Ende mit einer Spindel (31) gekoppelt ist, wobei die Spindel (31) über ein Außengewinde (32) mit einem Innengewinde (33) im Zug-/ Druckelement (9) in Wirkverbindung steht, um die Rotationsbewegung der Welle (30) in eine Axialbewegung des Zug-/ Druckelements (9) umzuwandeln.

11. Medizinisches Instrument nach Anspruch 9 oder 10, **dadurch gekennzeichnet, dass** die Welle (30) und die Spindel (31) über eine Verbindungshülse (34) miteinander gekoppelt sind.

12. Medizinisches Instrument nach einem der Ansprüche 9 bis 11, **dadurch gekennzeichnet, dass** die Welle (35) zum Verdrehen der Werkzeugspitze (8) sowie die Welle (30) zur Betätigung des Zug-/ Druckelements (18) koaxial zueinander im Schaft (2) gelagert sind.

13. Medizinisches Instrument nach einem der Ansprüche 9 bis 12, **dadurch gekennzeichnet, dass** sowohl die Welle (30) als auch die Welle (35) als flexible Wellen ausgebildet sind.

14. Medizinisches Instrument nach Anspruch 13, **dadurch gekennzeichnet, dass** in einem distalen Endbereich des Schaftes (2) ein in Richtung der Längsachse (7) des Schaftes (2) verlaufender und bis zum distalen Ende (14) des Schaftes (2) reichender Schlitz (38) ausgebildet ist, wobei der Schlitz (38) entlang der Seite des Schaftes (2) ausgebildet ist, die der Richtung der Abwinklung der Werkzeugspitze (8) gegenüber liegt, um ein radiales Austreten der Wellen (30 und 35) aus dem Schaft (2) beim Abwinkeln der Werkzeugspitze (8) zu ermöglichen.

15. Medizinisches Instrument nach einem der Ansprüche 10 bis 14, **dadurch gekennzeichnet, dass** die Welle (35) zum Verdrehen der Werkzeugspitze (8) und die Spindel (31) zum Betätigen des mindestens einen verschwenkbaren Maulteils (5 oder 6) des Werkzeugs (4) über einen koaxial auf der Spindel (31) angeordneten und radial nach außen wirkenden Spannring (37) relativ zueinander entkoppelt sind.

## Claims

1. Medical instrument with a hollow shaft (2), at the proximal end of which a handle (3) is arranged, and at the distal end of which a tool (4) with two jaw parts (5, 6) is arranged, of which at least one jaw part (5 or 6) is pivotable relative to the other jaw part (6 or 5), wherein a distal end region of the shaft (2) that carries the tool (4) is configured as a tool tip (8) that can be deflected with respect to the longitudinal axis (7) of the shaft (2), and the tool tip (8) is rotatable about the longitudinal axis (7) of the shaft (2) respectively about the longitudinal axis (7a) of the tool tip (8), wherein the rotation of the tool tip (8) about the longitudinal axis (7) of the shaft (2) respectively about the longitudinal axis (7a) of the tool tip (8) is effected via a first actuation element (10) which is mounted rotatably in the hollow shaft (2) and which is operatively connected at the proximal end to the handle (3), and wherein the deflection of the tool tip (8) is effected via a second actuation element (11) which is mounted axially displaceably in the hollow shaft (2) and which is operatively connected at the proximal end to the handle (3), wherein the at least one pivotable jaw part (5 or 6) of the tool (4) is adjustable between a closed position and an open position via a pull/push element (9) which is mounted axially displaceably in the tool tip (8) and which is operatively connected at the proximal end to the handle (3), and wherein the pull/push element (9) for the actuation of the at least one pivotable jaw part (5 or 6) is mounted eccentrically in the tool tip (8), parallel to the longitudinal axis (7) of the shaft (2) respectively the longitudinal axis (7a) of the tool tip (8), **characterized in that**
the tool tip (8) is mounted at the distal end (14) of the shaft (2) via a slotted-guide element (12) in such a way as to be deflectable with respect to the longitudinal axis (7) of the shaft (2), and **in that** the proximal end of the tool tip (8) is mounted in the slotted-guide element (12) so as to be rotatable about the longitudinal axis (7) of the shaft (2) respectively about the longitudinal axis (7a) of the tool tip (8) via a hollow pin (13) arranged eccentrically with respect to the longitudinal axis (7) of the shaft (2) respectively the longitudinal axis (7a) of the tool tip (8).

2. Medical instrument according to Claim 1, **characterized in that** the distal end (14) of the shaft (2) is shaped bifurcated and has two parallel webs (15) arranged at a distance from each other.

3. Medical instrument according to Claim 2, **characterized in that** the slotted-guide element (12) is mounted between the two webs (15) of the distal end (14) of the shaft (2) via bearing pins (16) so as to be deflectable with respect to the longitudinal axis (7) of the shaft (2), wherein the bearing pins (16) are mounted in corresponding bearing receptacles (17) of the webs (15).

4. Medical instrument according to one of Claims 1 to 3, **characterized in that** the second actuation element (11) mounted in the shaft (2) in order to deflect the tool tip (8) is configured as an axially displaceable pull/push element (18), which is mounted at the distal end on the slotted-guide element (12).

5. Medical instrument according to one of Claims 1 to 4, **characterized in that** the deflection of the tool tip (8) with respect to the longitudinal axis (7) of the shaft (2) can be limited via at least one stop element.

6. Medical instrument according to Claim 5, **characterized in that** the at least one stop element is configured as a pin (21) which is mounted in at least one web (15) of the distal end (14) of the shaft (2) and which engages in a guide track (22) formed in the slotted-guide element (12) .

7. Medical instrument according to one of Claims 1 to 6, **characterized in that** the first actuation element (10) for rotation of the tool tip (8) about the longitudinal axis (7) of the shaft (2) and/or the longitudinal axis (7a) of the tool tip (8) is configured as a shank (35) which is mounted rotatably in the shaft (2) and which is coupled at the distal end to the tool tip (8).

8. Medical instrument according to Claim 7, **characterized in that** the shank (35) is secured at the tool tip (8) via a connection sleeve (36).

9. Medical instrument according to one of Claims 1 to 8, **characterized in that** the pull/push element (9) mounted axially displaceably in the tool tip (8) and serving for actuation of the at least one pivotable jaw part (5 or 6) of the tool (4) can be driven via a shank (30) which is mounted rotatably in the shaft (2) and which is coupled at the proximal end to the handle (3).

10. Medical instrument according to Claim 9, **characterized in that** the shank (30) is coupled at its distal end to a spindle (31), wherein the spindle (31) is operatively connected by an external thread (32) to an internal thread (33) in the pull/push element (9), in order to convert the rotational movement of the shank (30) into an axial movement of the pull/push element (9).

11. Medical instrument according to Claim 9 or 10, **characterized in that** the shank (30) and the spindle (31) are coupled to each other via a connection sleeve (34).

12. Medical instrument according to one of Claims 9 to 11, **characterized in that** the shank (35) for rotation of the tool tip (8) and the shank (30) for actuation of the pull/push element (18) are mounted coaxially to each other in the shaft (2).

13. Medical instrument according to one of Claims 9 to 12, **characterized in that** the shank (30) and the shank (35) are both configured as flexible shanks.

14. Medical instrument according to Claim 13, **characterized in that** a slit (38) running in the direction of the longitudinal axis (7) of the shaft (2) and reaching as far as the distal end (14) of the shaft (2) is formed in a distal end region of the shaft (2), wherein the slit (38) is formed along that side of the shaft (2) which lies opposite the direction of the deflection of the tool tip (8), in order to allow the shanks (30 and 35) to emerge radially from the shaft (2) during the deflection of the tool tip (8).

15. Medical instrument according to one of Claims 10 to 14, **characterized in that** the shank (35) for rotation of the tool tip (8) and the spindle (31) for actuation of the at least one pivotable jaw part (5 or 6) of the tool (4) are decoupled relative to each other via a radially outwardly acting clamping ring (37), which is arranged coaxially on the spindle (31).

## Revendications

1. Instrument médical muni d'une tige creuse (2), à l'extrémité proximale de laquelle une poignée (3) est agencée et à l'extrémité distale de laquelle un outil (4) muni de deux parties de mâchoire (5, 6) est agencé, parmi lesquelles au moins une partie de mâchoire (5 ou 6) est pivotante relativement à l'autre partie de mâchoire (6 ou 5), une zone d'extrémité distale portant l'outil (4) de la tige (2) étant configurée en tant que pointe d'outil (8) pouvant être coudée par rapport à l'axe longitudinal (7) de la tige (2), et la pointe d'outil (8) étant rotative autour de l'axe longitudinal (7) de la tige (2) respectivement autour de l'axe longitudinal (7a) de la pointe d'outil (8), la mise en rotation de la pointe d'outil (8) autour de l'axe longitudinal (7) de la tige (2) respectivement autour de l'axe longitudinal (7a) de la pointe d'outil (8) ayant lieu par l'intermédiaire d'un premier élément d'actionnement (10) monté de manière rotative dans la tige creuse (2), qui est en liaison fonctionnelle côté proximal avec la poignée (3), et le coudage de la pointe d'outil (8) ayant lieu par l'intermédiaire d'un deuxième élément d'actionnement (11) monté de manière axialement coulissante dans la tige creuse (2) et qui se trouve en liaison fonctionnelle côté proximal avec la poignée (3), et l'au moins une partie de mâchoire pivotante (5 ou 6) de l'outil (4) étant réglable entre une position fermée et une position ouverte par l'intermédiaire d'un élément de traction/pression (9) monté de manière axialement coulissante dans la pointe d'outil (8), qui se trouve en liaison fonctionnelle côté proximal avec la poignée (3), l'élément de traction/pression (9) pour l'actionnement de l'au moins une partie de mâchoire pivotante (5 ou 6) étant monté de manière excentriquement parallèle à l'axe longitudinal (7) de la tige (2) respectivement à l'axe longitudinal (7a) de la pointe d'outil (8) dans la pointe d'outil (8),
**caractérisé en ce que**
la pointe d'outil (8) est montée à l'extrémité distale (14) de la tige (2) de manière à pouvoir être coudée par rapport à l'axe longitudinal (7) de la tige (2) par l'intermédiaire d'un élément coulisseau (12), et **en ce que** la pointe d'outil (8) est montée dans l'élément coulisseau (12) de manière à pouvoir être mise en rotation autour de l'axe longitudinal (7) de la tige (2) respectivement autour de l'axe longitudinal (7a) de la pointe d'outil (8) côté proximal par l'intermédiaire d'un tenon creux (13) agencé excentriquement par rapport à l'axe longitudinal (7) de la tige (2) respectivement l'axe longitudinal (7a) de la pointe d'outil (8).

2. Instrument médical selon la revendication 1, **caractérisé en ce que** l'extrémité distale (14) de la tige (2) est configurée fourchue et comprend deux entretoises (15) parallèles et espacées l'une de l'autre.

3. Instrument médical selon la revendication 2, **caractérisé en ce que** l'élément coulisseau (12) est monté entre les deux entretoises (15) de l'extrémité distale (14) de la tige (2) de manière à pouvoir être coudé par rapport à l'axe longitudinal (7) de la tige (2) par l'intermédiaire de pivots (16), les pivots (16) étant montés dans des logements de palier correspondants (17) des entretoises (15).

4. Instrument médical selon l'une quelconque des revendications 1 à 3, **caractérisé en ce que** le deuxième élément d'actionnement (11) monté dans la tige (2) pour le coudage de la pointe d'outil (8) est configuré en tant qu'élément de traction/pression axialement coulissant (18), qui est monté côté distal sur l'élément coulisseau (12).

5. Instrument médical selon l'une quelconque des revendications 1 à 4, **caractérisé en ce que** le coudage de la pointe d'outil (8) par rapport à l'axe longitudinal (7) de la tige (2) peut être limité par l'intermédiaire d'au moins un élément de butée.

6. Instrument médical selon la revendication 5, **caractérisé en ce que** l'au moins un élément de butée est configuré en tant que tenon (21) monté dans au moins une entretoise (15) de l'extrémité distale (14) de la tige (2), qui entre en prise dans une glissière (22) formée dans l'élément coulisseau (12).

7. Instrument médical selon l'une quelconque des revendications 1 à 6, **caractérisé en ce que** le premier élément d'actionnement (10) pour la mise en rotation de la pointe d'outil (8) autour de l'axe longitudinal (7) de la tige (2) ou de l'axe longitudinal (7a) de la pointe d'outil (8) est configuré en tant qu'arbre (35) monté de manière à pouvoir être mis en rotation dans la tige (2), qui est couplé côté distal à la pointe d'outil (8).

8. Instrument médical selon la revendication 7, **caractérisé en ce que** l'arbre (35) est fixé à la pointe d'outil (8) par l'intermédiaire d'une douille de liaison (36).

9. Instrument médical selon l'une quelconque des revendications 1 à 8, **caractérisé en ce que** l'élément de traction/pression (9) monté de manière axialement coulissante dans la pointe d'outil (8) pour l'actionnement de l'au moins une partie de mâchoire pivotante (5 ou 6) de l'outil (4) peut être entraîné par l'intermédiaire d'un arbre (30) monté de manière à pouvoir être mis en rotation dans la tige (2), qui est couplé côté proximal à la poignée (3).

10. Instrument médical selon la revendication 9, **caractérisé en ce que** l'arbre (30) est couplé à une broche (31) à son extrémité distale, la broche (31) se trouvant en liaison fonctionnelle par l'intermédiaire d'un filetage extérieur (32) avec un filetage intérieur (33) dans l'élément de traction/pression (9), afin de transformer le mouvement de rotation de l'arbre (30) en un mouvement axial de l'élément de traction/pression (9) .

11. Instrument médical selon la revendication 9 ou 10, **caractérisé en ce que** l'arbre (30) et la broche (31) sont couplés l'un à l'autre par l'intermédiaire d'une douille de liaison (34).

12. Instrument médical selon l'une quelconque des revendications 9 à 11, **caractérisé en ce que** l'arbre (35) pour la mise en rotation de la pointe d'outil (8), ainsi que l'arbre (30) pour l'actionnement de l'élément de traction/pression (18) sont montés coaxialement l'un de l'autre dans la tige (2).

13. Instrument médical selon l'une quelconque des revendications 9 à 12, **caractérisé en ce qu'**aussi bien l'arbre (30) que l'arbre (35) sont configurés en tant qu'arbres flexibles.

14. Instrument médical selon la revendication 13, **caractérisé en ce qu'**une fente (38) s'étendant dans la direction de l'axe longitudinal (7) de la tige (2) et atteignant l'extrémité distale (14) de la tige (2) est formée dans une zone d'extrémité distale de la tige (2), la fente (38) étant formée le long du côté de la tige (2) qui est opposé à la direction du coudage de la pointe d'outil (8), afin de permettre une sortie radiale des arbres (30 et 35) de la tige (2) lors du coudage de la pointe d'outil (8).

15. Instrument médical selon l'une quelconque des revendications 10 à 14, **caractérisé en ce que** l'arbre (35) pour la mise en rotation de la pointe d'outil (8) et la broche (31) pour l'actionnement de l'au moins une partie de mâchoire pivotante (5 ou 6) de l'outil (4) sont découplés l'un de l'autre par l'intermédiaire d'une bague de serrage (37) agencée coaxialement sur la broche (31) et agissant radialement vers l'extérieur.
